# EUROPEAN PATENT APPLICATION

(11) **EP 3 988 934 A1**
(43) Date of publication of application: **27.04.2022**
(21) Application number: 20203017.7
(22) Date of filing: 21.10.2020
(51) Int. Cl.: G01N 33/497, G01N 33/68, A61B 5/08, A61B 5/00

(54) **BIOMARKERS IN EXHALED BREATH FOR DIAGNOSING A MAJOR DEPRESSIVE DISORDER**

(71) Applicant: Otto-von-Guericke-Universität Magdeburg, 39106 Magdeburg (DE)
(72) Inventor: Frodl, Thomas, 39108 Magdeburg (DE); Hoeschen, Christoph, 39114 Magdeburg (DE); Lüno, Marian, 39116 Magdeburg (DE); Meyer-Lotz, Gabriela, 39116 Magdeburg (DE); Gbaoui, Laila, 12355 Berlin (DE)

(57) **Abstract**

The present invention relates to a method of diagnosing a major depressive disorder (MDD) in a patient. Further, the present invention relates to a method of determining the course of a MDD in a patient. Furthermore, the present invention relates to a method of determining whether a patient responds to a therapeutic treatment of a MDD. In addition, the present invention relates to a device which can be used in these methods. Moreover, the present invention relates to a kit comprising the device.

## Description

The present invention relates to a method of diagnosing a major depressive disorder (MDD) in a patient. Further, the present invention relates to a method of determining the course of a MDD in a patient. Furthermore, the present invention relates to a method of determining whether a patient responds to a therapeutic treatment of a MDD. In addition, the present invention relates to a device which can be used in these methods. Moreover, the present invention relates to a kit comprising the device.

### BACKGROUND OF THE INVENTION

Brain disorders cost Europe almost € 800 billion (US $1 trillion) a year - more than cancer, cardiovascular disease and diabetes do cost together. One of the most common psychiatric disorders is a major depressive disorder (MDD) that can effectively be treated with psychotherapy and/or antidepressants acting at serotonergic, noradrenergic and nowadays glutamatergic neurotransmission. However, still one third of patients do not respond to at least two different antidepressant trials and might need as early as possible different treatment options.

Unfortunately, there are currently no non-invasive, easy, and frequently applicable biomarkers available that could help to facilitate diagnosis of a depressive episode or that could guide to the most effective therapy. Noteworthy, currently there are studies on biomarkers in the area of MDD using e.g. neuroimaging, inflammation, hormones, oxidative stress and genetics. Significant differences were described for MDD compared to healthy controls, e.g. for functional and structural neuroimaging, immune system and inflammation. Moreover, the dysregulation of the hypothalamic-pituitary-adrenal (HPA) hormone axis has well been described in MDD. In a recent meta-analysis on prospective studies that aimed to predict MDD onset, relapse and recurrence by imaging, genetic, inflammation and hormone markers, only cortisol was detected to be a significant predictor (Kennis *et al.*, 2020) and thus, signatures related to the cortisol/stress-system are of relevance. With respect to cortisol, the cortisol awakening response (CAR) is one of the most straightforward measures of dynamics of the HPA axis and can be obtained reliably (Kudielka *et al.*, 2007). The CAR is thought to reflect the sensitivity of the HPA axis to the natural challenge awakening. However, overall, no widely accepted clinical biomarkers have been developed.

The methods presently available are invasive blood tests and it is not convenient for patients to take blood samples continuously throughout the day or even over several days in order to follow the disease longitudinally. Moreover, functional neuroimaging is expensive and time consuming and it is not feasible to scan patients several times as a routine procedure. A non-invasive longitudinal measurement would certainly increase the information on individual disease characteristics in patients. Thus, new non-invasive biomarkers are highly needed.

The present inventors have chosen the breath gas analysis in order to identify new non-invasive biomarkers indicative for a MDD. By comparing the breath gas of patients suffering from a MDD and healthy control subjects, the present inventors surprisingly found that the exhaled breath gas includes volatile organic compounds (VOCs) that are exhaled shortly after their production and are indicative for the presence of a MDD. Because the lungs act as an exchange system for gas between the internal system and external environment, the internal system in disorders like MDD can be assessed through the analysis of the volatile organic compounds in the exhaled breath. These breath biomarkers are non-invasive and easy to determine. They allow the diagnosis of a MDD. These breath biomarkers can also be used to predict short-term disease changes. Using these breath biomarkers, effects of antidepressant therapy can further be examined and, thus, therapy success can be monitored.

### SUMMARY OF THE INVENTION

In a first aspect, the present invention relates to a method of diagnosing a major depressive disorder (MDD) in a patient (suspected of having a MDD) comprising the step of:
determining the level of at least one compound in a sample of exhaled breath obtained from a patient (suspected of having a MDD),
wherein the at least one compound is selected from the group consisting of methylguanidine, lactic acid, butyric acid, tetrahydro-tiaphene, putrescine, acetamide, isoprene, toluene, and acetonitrile.

In a second aspect, the present invention relates to a method of determining the course of a major depressive disorder (MDD) in a patient (having a MDD) comprising the step of: determining the level of at least one compound in a sample of exhaled breath obtained from a patient (having a MDD),
wherein the at least one compound is selected from the group consisting of methylguanidine, lactic acid, butyric acid, tetrahydro-tiaphene, putrescine, acetamide, isoprene, toluene, and acetonitrile.

In a third aspect, the present invention relates to a method of determining whether a patient (suffering from a MDD) responds to a therapeutic treatment of a major depressive disorder (MDD) comprising the step of:
determining the level of at least one compound in a sample of exhaled breath obtained from a patient (suffering from a MDD),
wherein the at least one compound is selected from the group consisting of methylguanidine, lactic acid, butyric acid, tetrahydro-tiaphene, putrescine, acetamide, isoprene, toluene, and acetonitrile.

In a fourth aspect, the present invention relates to the use of at least one compound for diagnosing a major depressive disorder (MDD) in a patient (suspected of having a MDD), for determining the course of a major depressive disorder (MDD) in a patient (having a MDD), or for determining whether a patient (suffering from a MDD) responds to a therapeutic treatment of a MDD, wherein the at least one compound is selected from the group consisting of methylguanidine, lactic acid, butyric acid, tetrahydro-tiaphene, putrescine, acetamide, isoprene, toluene, and acetonitrile.

In a fifth aspect, the present invention relates to a device comprising
(i) an inlet for receiving exhaled breath, and
(ii) an analysis unit configured to analyze the exhaled breath,
wherein the analysis comprises the determination of the level of at least one compound in a sample of exhaled breath obtained from a patient, and
wherein the at least one compound is selected from the group consisting of methylguanidine, lactic acid, butyric acid, tetrahydro-tiaphene, putrescine, acetamide, isoprene, toluene, and acetonitrile.

In a sixth aspect, the present invention relates to a kit comprising the device of the fifth aspect.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "major depressive disorder (MDD)", as used herein, refers to a mental disorder characterized by at least two weeks of pervasive low mood. Low self-esteem, loss of interest in normally enjoyable activities, low energy, and pain without a clear cause are common symptoms. Those affected may also occasionally have delusions or hallucinations. Some patients have periods of depression separated by years, while others nearly always have symptoms present. Major depression is more severe and lasts longer than sadness, which is a normal part of life. A major depressive disorder can also be designated as depression.
The term "major depressive disorder (MDD)", as used herein, includes melancholic depression, atypical depression, catatonic depression, depression with anxious distress, depression with peri-partum onset, post-partum onset, and seasonal affective disorder.
The diagnosis of a major depressive disorder is based on the person's reported experiences, the psychiatric history, a mental status examination and a physical examination. There is presently no laboratory test for the disorder, but testing may be done to rule out physical conditions that can cause similar symptoms. The present inventors are the first who have identified biomarkers allowing the diagnosis of a MDD in exhaled breath of a patient.
Those with major depressive disorder are typically treated with counseling and antidepressant medication. Medication appears to be effective, but the effect may only be significant in the most severely depression. Types of counseling used include cognitive behavioral therapy (CBT) and interpersonal therapy. Electroconvulsive therapy (ECT) may also be considered. Hospitalization may be necessary in cases with a risk of harm to self and may occasionally occur against a person's wishes.

The term "diagnosing a major depressive disorder (MDD)", as used herein, means determining whether a patient shows signs of or suffers from a MDD.

The term "determining the course of a major depressive disorder (MDD)", as used herein, means determining the development of a MDD over time, e.g. whether a MDD worsens in the patient, does not worsen/is stable/not progressing in the patient, or improves in the patient over time.

The term "determining whether a patient responds to a therapeutic treatment of a MDD", as used herein, means determining whether a therapeutic treatment is effective or not, e.g. whether the dose of a drug has to be increased or decreased or whether the drug has to be changed.

The term "Diagnostic and Statistical Manual of Mental Disorders-V (DSM-V)", as used herein, refers to the fifth update to the Diagnostic and Statistical Manual of Mental Disorders, the taxonomic and diagnostic tool published by the American Psychiatric Association (APA). In the United States, the DSM serves as the principal authority for psychiatric diagnoses. Treatment recommendations, as well as payment by health care providers, are often determined by DSM classifications, so the appearance of a new version has significant practical importance.

The term "patient", as used herein, refers to any subject for whom it is desired to know whether she or he suffers from a major depressive disorder (MDD). In particular, the term "patient", as used herein, refers to a subject suspected to be affected by a MDD. The patient may be diagnosed to be affected by a MDD, i.e. diseased, or may be diagnosed to be not affected by a MDD, i.e. healthy. The term "patient", as used herein, also refers to a subject which is affected by a MDD, i.e. diseased. The patient may be retested for a MDD and may be diagnosed to be still affected by a MDD, i.e. diseased, or not affected by a MDD anymore, i.e. healthy, for example after therapeutic intervention. The patient may also be retested for a MDD and may be diagnosed as having developed an advanced form of a MDD.
It should be noted that a patient that is diagnosed as being healthy, i.e. not suffering from a MDD, may possibly suffer from another disease not tested/known.
The patient may be any mammal, including both a human and another mammal, e.g. an animal. Human patients are particularly preferred.

The term "(control) subject", as used herein, refers to a subject known to be affected by a major depressive disorder (MDD) (positive control), i.e. diseased. Said (control) subject may have developed an advanced form of a MDD. The term "(control) subject", as used herein, also refers to a subject known to be not affected by a MDD (negative control), i.e. healthy. Thus, the term "healthy subject", as used herein, means a subject which is known to be not affected by a MDD.
It should be noted that a (control) subject which is known to be healthy, i.e. not suffering from MDD, may possibly suffer from another disease not tested/known.
The (control) subject may be any mammal, including both a human and another mammal, e.g. an animal. Human (control) subjects are particularly preferred.

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease.
The treatment of a MDD includes, but is not limited to, administration of a drug, psychotherapy, exercise training, cognitive training, and physical rehabilitation. Specific therapy forms are the cognitive behavioral therapy (CBT), interpersonal therapy, and electroconvulsive therapy (ECT). The drug is preferably an antidepressant. The antidepressant is more preferably selected from the group consisting of citalopram, escitalopram, paroxetine, sertraline, fluoxetine, venlafaxine, duloxetine, milnaciprame, bupropion, and mirtazapine.

The term "breathomics", as used herein, refers to the study of compounds found in exhaled air. The field of breathomics mainly focuses on health-related volatile organic compounds (VOCs). Since the amount of these compounds varies with health status, breathomics holds great promise to deliver non-invasive diagnostic tools. Thus, the main aim of breathomics is to find patterns of VOCs related to abnormal metabolic processes occurring in the human or animal body. The origin of breath VOCs include the environment (termed exogenous), the host (endogenous), and also the microbiome (the microorganisms that inhabit the mouth, lung and gut). Exhaled breath is predominantly composed of nitrogen, oxygen, carbon dioxide, argon as well as water vapour, whereas the volatile organic compounds (VOCs) which may be diagnostically useful are only found in low concentrations. However, modern analytical instruments made the identification of VOCs patterns via human or animal olfaction for disease diagnosis more reliable and robust. The advantage of breath diagnosis is its non-invasive nature, with the ability to take repeat measurements with little stress or discomfort to the individual under investigation.
The present inventors surprisingly found compounds which are deregulated between patients suffering from a major depressive disorder (MDD) and healthy subjects. Said compounds are selected from the group consisting of methylguanidine, lactic acid, butyric acid, tetrahydro-tiaphene, putrescine, acetamide, isoprene, toluene, and acetonitrile. While methylguanidine, lactic acid, butyric acid, tetrahydro-tiaphene, putrescine, acetamide, and isoprene are metabolites, toluene and acetonitrile are markers for toxicity found in the environment. The compounds identified by the present inventors can also be designated as volatile organic compounds (VOCs). While methylguanidine, lactic acid, butyric acid, tetrahydro-tiaphene, putrescine, acetamide, and isoprene are endogenous VOCs, toluene and acetonitrile are exogenous VOCs.

The term "sample of exhaled breath", as used herein, refers to gas sample derived from the body of a patient or (control) subject containing the at least one compound selected from the group consisting of methylguanidine, lactic acid, butyric acid, tetrahydro-tiaphene, putrescine, acetamide, isoprene, toluene, and acetonitrile.
The gas sample derived from the body of a patient or (control) subject encompasses exhaled condensate and exhaled gas. If the gas sample is obtained from patients to be tested, it is simply designated as "sample of exhaled breath". If the gas sample is obtained from control subjects, it is designated as "reference sample of exhaled breath".

The term "sensitivity", as used herein, refers to the number of true positive patients (%) with regard to the number of all patients (100%). The patients suffer from MDD. The sensitivity is calculated by the following formula: Sensitivity= TP/(TP+FN) (TP= true positives; FN=false negatives).

The term "specificity", as used herein, relates to the number of true negative individuals (%) with regard to the number of all healthy subjects (100%). The specificity is calculated by the following formula: Specificity= TN/(TN+FP) (TN= true negatives; FP=false positives).

The term "accuracy", as used herein, means a statistical measure for the correctness of classification or identification of sample types. The accuracy is the proportion of true results (both true positives and true negatives).

The term "AUC", as used herein, relates to an abbreviation for the area under a curve. In particular, it refers to the area under a Receiver Operating Characteristic (ROC) curve. The term "Receiver Operating Characteristic (ROC) curve", as used herein, refers to a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve is a measure for the accuracy of a diagnostic test (the larger the area the better, optimum is 1, a random test would have a ROC curve lying on the diagonal with an area of 0.5 (see, for reference, for example, JP. Egan. Signal Detection Theory and ROC Analysis).

The term "level", as used herein, refers to an amount (measured for example in grams, mole, or ion counts) or concentration (e.g. absolute or relative concentration) of the at least one compound selected from the group consisting of methylguanidine, lactic acid, butyric acid, tetrahydro-tiaphene, putrescine, acetamide, isoprene, toluene, and acetonitrile.
The term "level", as used herein, also comprises scaled, normalized, or scaled and normalized amounts or values. The level may also be a cut-off level. The level may further be an average level. It may be determined by measuring levels and calculating the "average" amount or value (e.g. mean, median or modal amount or value) thereof.
If the level is determined in an exhaled breath sample obtained from patients which are to be tested, it is simply designated as "level". If the level is determined in an exhaled breath sample obtained from control subjects, it is designated as "reference level".

The level of the at least one compound is determined by spectrometry or chromatography. The spectrometry is specifically mass spectrometry (MS). The chromatography is specifically liquid chromatography (LC) or affinity chromatography.
The term "mass spectrometry (MS)", as used herein, refers to the use of an ionization source to generate gas phase ions from a sample on a surface and detecting the gas phase ions with a mass spectrometer. The mass spectrometry is preferably selected from the group consisting of proton-transfer-reaction mass spectrometry (PTR-MS), time of flight mass spectrometry (TOF-MS), and quadrupole mass spectrometry (QMS).
The general concept of PTR-MS is to bring the breath gas into a drift tube. For this purpose, the gas may be mixed and thus react with protons (H₃O⁺ - ions). The protonated VOCs will then be analyzed by the mass spectrometry part. Typically, masses between m/z 19 and 200 can be analyzed with the quadrupole based analyzer unit and masses between m/z 19 and about 800 can be analyzed with the TOF based analyzer. The linear range of the system is between a few volume parts per trillion (pptv) and a few hundred parts per billion (ppbv).

The term "liquid chromatography (LC)", as used herein, refers to a separation technique in which the mobile phase is a liquid. It can be carried out either in a column or a plane. A liquid chromatography that generally utilizes very small packing particles and a relatively high pressure is referred to as high performance liquid chromatography (HPLC). In HPLC, the sample is forced by a liquid at high pressure (the mobile phase) through a column that is packed with a stationary phase composed of irregularly or spherically shaped particles, a porous monolithic layer, or a porous membrane.

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of patient care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the patient. This increases the likelihood that the patient, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the patient so that the treatment plan can be adjusted as necessary before the patient leaves.

### Embodiments of the invention

The present inventors have chosen the breath gas analysis in order to identify new non-invasive biomarkers indicative for a major depressive disorder (MDD). By comparing the breath gas of patients suffering from a MDD and healthy control subjects, the present inventors surprisingly found that the exhaled breath gas includes volatile organic compounds (VOCs) that are exhaled shortly after their production and are indicative for the presence of a MDD. Because the lungs act as an exchange system for gas between the internal system and external environment, the internal system in disorders like MDD can be assessed through the analysis of the volatile organic compounds in the exhaled breath. These breath biomarkers are non-invasive and easy to determine. They allow the diagnosis of a MDD. These breath biomarkers can also be used to predict short-term disease changes. Using these breath biomarkers, effects of antidepressant therapy can further be examined and, thus, therapy success can be monitored.

Thus, in a first aspect, the present invention relates to a method of diagnosing a major depressive disorder (MDD) in a patient (suspected of having a MDD) comprising the step of:
determining the level of at least one compound (e.g. at least 1, 2, 3, 4, 5, 6, 7, 8, or 9 compound(s)) in a sample of exhaled breath obtained from a patient (suspected of having a MDD),
wherein the at least one compound is selected from the group consisting of methylguanidine, lactic acid, butyric acid, tetrahydro-tiaphene, putrescine, acetamide, isoprene, toluene, and acetonitrile.

In one embodiment, the level of the at least one compound is compared to a reference level of said at least one compound. Thus, in one particular embodiment, the present invention relates to a method of diagnosing a major depressive disorder (MDD) in a patient (suspected of having a MDD) which comprises the steps of:
(i) determining the level of at least one compound (e.g. at least 1, 2, 3, 4, 5, 6, 7, 8, or 9 compound(s)) in a sample of exhaled breath obtained from a patient (suspected of having a MDD), and
(ii) comparing the level of the at least one compound to a reference level of said at least one compound,
wherein the at least one compound is selected from the group consisting of methylguanidine, lactic acid, butyric acid, tetrahydro-tiaphene, putrescine, acetamide, isoprene, toluene, and acetonitrile.
The above comparison allows to diagnose a MDD in the patient suspected of having a MDD. The patient may be diagnosed as suffering from a MDD, i.e. being diseased, or as not suffering from a MDD, i.e. being healthy.

The reference level may be any level which allows to determine whether a patient suffers from a MDD or not. It is preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one healthy subject, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference samples of exhaled breath obtained from between 2 and 500 healthy subjects. It is even more preferred that the reference level is determined by measuring between 50 and 500 reference samples of exhaled breath obtained from between 50 and 500 healthy subjects. It is most preferred that the reference level is determined by measuring between 100 and 500 reference samples of exhaled breath obtained from between 100 and 500 healthy subjects.

It is practicable to take one reference sample of exhaled breath per subject for analysis. If additional reference samples of exhaled breath are required, such as to determine the reference level in different reference samples of exhaled breath (e.g. at different points in time), the same subject may be (re)tested. Said reference level may be an average reference level. It may be determined by measuring reference levels and calculating the "average" value (e.g. mean, median or modal value) thereof.

As mentioned above, the level of the at least one compound is compared to a reference level of said at least one compound. Said reference level is the level determined by measuring a reference sample of exhaled breath. For example, if the level of lactic acid is determined in a sample of exhaled breath obtained from a patient, it is compared to a reference level of lactic acid determined in a reference sample of exhaled breath. Alternatively, if the level of lactic acid and the level of butyric acid are determined in a sample of exhaled breath obtained from a patient, both levels are compared to the respective reference levels, i.e. the level of lactic acid is compared to the reference level of lactic acid and the level of butyric acid is compared to the reference level of butyric acid determined in a reference sample of exhaled breath.

It is further (additionally or alternatively) preferred that the level of the at least one compound selected from the group consisting of methylguanidine, lactic acid, butyric acid, tetrahydro-tiaphene, putrescine, acetamide, and isoprene below the reference level indicates that the patient has a MDD, and/or
the level of the at least one compound selected from the group consisting of toluene and acetonitrile above the reference level indicates that the patient has a MDD.
In particular, the level of the at least one compound is at least 0.5-fold, more preferably at least 1-fold, even more preferably at least 1.5-fold, still more preferably at least 2-fold, and most preferably at least 3-fold below/above the reference level. For example, the level of the at least one compound is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

In principle, the determination of the level of the at least one compound can take place at any time of day. However, the present inventors found that the time after awakening/getting up is particularly suitable for breath gas analysis. Thus, it is also (additionally or alternatively) preferred that the level of the at least one compound is determined in a sample of exhaled breath obtained from a patient (suspected of having a MDD) at a point in time within 0 and 5 minutes after awakening (= at awakening or immediately after awakening), at a point in time within < 5 minutes and 30 minutes after awakening, or at a point in time within < 30 minutes and 60 minutes after awakening.
In particular, the level of the at least one compound is determined in a sample of exhaled breath obtained from a patient (suspected of having a MDD) about 2 minutes (± 1 minute) after awakening, 30 minutes after awakening, or 60 minutes after awakening.
It is more preferred that the level of the at least one compound represents an average level of levels determined in a sample of exhaled breath obtained from a patient (suspected of having a MDD) at a point in time within 0 and 5 minutes after awakening (= at awakening or immediately after awakening), at a point in time within < 5 minutes and 30 minutes after awakening, and at a point in time within < 30 minutes and 60 minutes after awakening (= baseline level).
For example, the level of the at least one compound (e.g. lactic acid) is determined in a sample of exhaled breath obtained from a patient at a point in time within 0 and 5 minutes after awakening, the level of the (same) at least one compound (e.g. lactic acid) is determined in a sample of exhaled breath obtained from the (same) patient at a point in time within < 5 minutes and 30 minutes after awakening, and the level of the (same) at least one compound (e.g. lactic acid) is determined in a sample of exhaled breath obtained from the (same) patient at a point in time within < 30 minutes and 60 minutes after awakening (= baseline level). Said levels are then used to calculate an average value (e.g. mean, median or modal value).
In particular, the level of the at least one compound represents an average level of levels determined in a sample of exhaled breath obtained from a patient suspected of having a MDD about 2 minutes (± 1 minute) after awakening, 30 minutes after awakening, and 60 minutes after awakening (= baseline level).
As mentioned above, the level is compared to a reference level. In this respect, it should be noted that the reference level of the control subject is preferably determined/calculated in the same way as the level of the patient to be tested. If, for example, an average level is determined, this level is also compared to average reference level.

In a second aspect, the present invention relates to a method of determining the course of a major depressive disorder (MDD) in a patient (having a MDD) comprising the step of: determining the level of at least one compound (e.g. at least 1, 2, 3, 4, 5, 6, 7, 8, or 9 compound(s)) in a sample of exhaled breath obtained from a patient (having a MDD), wherein the at least one compound is selected from the group consisting of methylguanidine, lactic acid, butyric acid, tetrahydro-tiaphene, putrescine, acetamide, isoprene, toluene, and acetonitrile.

In one embodiment, the level of the at least one compound is compared to a reference level of said at least one compound. Thus, in one particular embodiment, the present invention relates to a method of determining the course of a major depressive disorder (MDD) in a patient (having a MDD) which comprises the steps of:
(i) determining the level of at least one compound (e.g. at least 1, 2, 3, 4, 5, 6, 7, 8, or 9 compound(s)) in a sample of exhaled breath obtained from a patient (having a MDD), and
(ii) comparing the level of the at least one compound to a reference level of said at least one compound,
wherein the at least one compound is selected from the group consisting of methylguanidine, lactic acid, butyric acid, tetrahydro-tiaphene, putrescine, acetamide, isoprene, toluene, and acetonitrile.
The above comparison allows to determine the course of a MDD in the patient having a MDD. It may be determined that a MDD worsens in the patient, that a MDD does not worsen/is stable/is not progressing in the patient, or that a MDD improves in the patient.

The reference level may be any level which allows to determine the course of a MDD in the patient. It is preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one subject having a MDD, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 subject(s) having a MDD. It is more preferred that the reference level is the level determined by measuring between 2 and 500 reference samples of exhaled breath obtained from between 2 and 500 subjects having a MDD. It is even more preferred that the reference level is determined by measuring between 50 and 500 reference samples of exhaled breath obtained from between 50 and 500 subjects having a MDD. It is most preferred that the reference level is determined by measuring between 100 and 500 reference samples of exhaled breath obtained from between 100 and 500 subjects having a MDD.

It is practicable to take one reference sample of exhaled breath per subject for analysis. If additional reference samples of exhaled breath are required, such as to determine the reference level in different reference samples of exhaled breath (e.g. at different points in time), the same subject may be (re)tested. Said reference level may be an average reference level. It may be determined by measuring reference levels and calculating the "average" value (e.g. mean, median or modal value) thereof.

In a specific embodiment, the reference level represents a threshold which allows to divide/separate the patients being sick from the patients being on the way to improvement/recovery.

A level above or below the reference level may indicate that a MDD worsens in the patient, a level comparable with the reference level may indicate that a MDD is unchanged in the patient, or a level above or below the reference level may indicate that a MDD improves in the patient.

In one preferred embodiment, the level of the at least one compound selected from the group consisting of methylguanidine, lactic acid, butyric acid, tetrahydro-tiaphene, putrescine, acetamide, and isoprene which is
(i) below the reference level indicates that MDD worsens in the patient,
(ii) comparable with the reference level indicates that MDD does not worsen/is stable/non-progressing in the patient, or
(iii) above the reference level indicates that MDD improves in the patient.

In one another preferred embodiment, the level of the at least one compound selected from the group consisting of toluene and acetonitrile which is
(i) above the reference level indicates that MDD worsens in the patient,
(ii) comparable with the reference level indicates that MDD does not worsen/is stable/non-progressing in the patient, or
(iii) below the reference level indicates that MDD improves in the patient.
In particular, the level of the at least one compound is at least 0.5-fold, more preferably at least 1-fold, even more preferably at least 1.5-fold, still more preferably at least 2-fold, and most preferably at least 3-fold below/above the reference level. For example, the level of the at least one compound is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.
"Not worsening", "stable" or "non-progressing", as mentioned above, may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Not worsening", "stable" or "non-progressing" in this respect may also mean that the detected level variation is within the accuracy of a measurement. The accuracy of a measurement depends on the measurement method used. Preferably, the level is constant over time.

In one alternative or additional embodiment, said determining comprises determining the level of the at least one compound in a sample of exhaled breath obtained from a patient (having a MDD) at a first point in time and in at least one further sample of exhaled breath obtained from the (same) patient at a later point in time and comparing said levels determined at the different time points. Thus, in one particular embodiment, the present invention relates to a method of determining the course of a major depressive disorder (MDD) in a patient (having a MDD) which comprises the steps of:
(i) determining the level of the at least one compound (e.g. at least 1, 2, 3, 4, 5, 6, 7, 8, or 9 compound(s)) in a sample of exhaled breath obtained from a patient (having a MDD) at a first point in time and in at least one further sample of exhaled breath obtained from the (same) patient at a later point in time, and
(ii) comparing the levels determined at the different time points,
wherein the at least one compound is selected from the group consisting of methylguanidine, lactic acid, butyric acid, tetrahydro-tiaphene, putrescine, acetamide, isoprene, toluene, and acetonitrile.

A level above or below the reference level may indicate that a MDD worsens in the patient, a level comparable with the reference level may indicate that a MDD is unchanged in the patient, or a level above or below the reference level may indicate that a MDD improves in the patient.

In one preferred embodiment,
the level of the at least one compound selected from the group consisting of methylguanidine, lactic acid, butyric acid, tetrahydro-tiaphene, putrescine, acetamide, and isoprene which
(i) decreases over time indicates that the MDD worsens in the patient,
(ii) does not change over time indicates that the MDD does not worsen/is stable/non-progressing in the patient, or
(iii) increases over time indicates that the MDD improves in the patient.

In one another preferred embodiment,
the level of the at least one compound selected from the group consisting of toluene and acetonitrile which
(i) increases over time indicates that the MDD worsens in the patient,
(ii) does not change over time indicates that the MDD does not worsen/is stable/non-progressing in the patient, or
(iii) decreases over time indicates that the MDD improves in the patient.
   "Not worsening", "stable" or "non-progressing", as mentioned above, may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Not worsening", "stable" or "non-progressing" in this respect may also mean that the detected level variation is within the accuracy of a measurement. The accuracy of a measurement depends on the measurement method used. Preferably, the level is constant over time.

It is preferred that the time period between the first point in time and the later point(s) in time amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, or at least 2 months. For example, the patient may be routinely checked, e.g. every day, every two weeks, every month. The patient may be (re)tested at 2, 3, 4, 5, 6 7, 8, 9, or 10 time points (first point in time and further point(s) in time).
The later point in time can also be designated as second, third, fourth, fifth, or sixth point in time. In a specific example, the time period between the first point in time and the second point in time amounts to 2 weeks, the time period between the second point in time and the third point in time amounts to 2 weeks, the time period between the third and fourth point in time amounts to 1 month, and the time period between each further point in time amounts to 1 month.

It is further preferred that the first point in time is within 0 and 5 minutes after awakening (= at awakening or immediately after awakening), within < 5 minutes and 30 minutes after awakening, or within < 30 minutes and 60 minutes after awakening.
In particular, the first point in time is about 2 minutes (± 1 minute) after awakening, 30 minutes after awakening, or 60 minutes after awakening.
The patient is then retested every two weeks over a time period of between one and two months and afterwards every month (first point in time and further point(s) in time).
The individual may be (re)tested at 2, 3, 4, 5, 6 7, 8, 9, or 10 time points (first point in time and further point(s) in time).

It is more preferred that a baseline level (as a first point in time) is first determined by determining the level in a sample of exhaled breath obtained from a patient having a MDD within 0 and 5 minutes after awakening (= at awakening or immediately after awakening), within < 5 minutes and 30 minutes after awakening, and within < 30 minutes and 60 minutes after awakening and calculating an average level of the levels determined at the different time points.
In particular, a baseline level (as a first point in time) is first determined by determining the level in a sample of exhaled breath obtained from a patient having a MDD about 2 minutes (± 1 minute) after awakening, 30 minutes after awakening, and 60 minutes after awakening and calculating an average level of the levels determined at the different time points.
This baseline level is then further compared with a level determined at a later point in time. For example, after the determination of the baseline level, the patient is retested every two weeks over a time period of between one and two months and afterwards every month (first point in time and further point(s) in time). The patient may be (re)tested at 2, 3, 4, 5, 6 7, 8, 9, or 10 time points (first point in time and further point(s) in time).
The later point in time can also be designated as second, third, fourth, fifth, or sixth point in time. In a specific example, a baseline level (as a first point in time), as described above, is first determined. The time period between the first point in time and the second point in time then amounts to 2 weeks, the time period between the second point in time and the third point in time amounts to 2 weeks, the time period between the third and fourth point in time amounts to 1 month, and the time period between each further point in time amounts to 1 month.

As mentioned above, the level is compared to a reference level. In this respect, it should be noted that the reference level of the control subject is preferably determined/calculated in the same way as the level of the patient to be tested. If, for example, an average level is determined, this level is also compared to average reference level.

In addition to the determination of the course of a MDD, the treatment of this disease can be monitored. It is namely preferred that the patient receives, has received, or had received a treatment, in particular therapeutic treatment, of a MDD during the determination of the course of a MDD. The treatment of a MDD is preferably selected from the group consisting of the administration of a drug, psychotherapy, exercise training, cognitive training, and physical rehabilitation.

In a third aspect, the present invention relates to a method of determining whether a patient (suffering from a MDD) responds to a therapeutic treatment of a major depressive disorder (MDD) comprising the step of:
determining the level of at least one compound in a sample of exhaled breath obtained from a patient (suffering from a MDD),
wherein the at least one compound is selected from the group consisting of methylguanidine, lactic acid, butyric acid, tetrahydro-tiaphene, putrescine, acetamide, isoprene, toluene, and acetonitrile.

It is preferred that the patient is a patient to whom at least once (1, 2, or 3 times) a drug to be used in said therapeutic treatment is administered, has been administered, or had been administered, and/or to whom at least once (1, 2, or 3 times) another form of therapeutic treatment (than the administration of a drug) is administered, has been administered, or had been administered. Usually, it takes at least 2 to 3 weeks before it can be determined whether the treatment is effective. During this time period, the patient has already received an antidepressant 14 to 21 times. Thus, the term "at least once" covers the first treatment and any further treatment which is required.
The way of administration may be oral, nasal, rectal, parenteral, vaginal, or topical. Parental administration includes subcutaneous, intracutaneous, intramuscular, intravenous or intraperitoneal administration.

It is further preferred that the sample of exhaled breath is obtained from the patient after at least the first (e.g. first, second, or third) administration of said drug and/or after at least the first (e.g. first, second, or third) administration of another form of therapeutic treatment (than the administration of a drug).
It is particularly preferred that the sample of exhaled breath is obtained from the patient in a time period of between 3 months and 1 day after at least the first (e.g. first, second, or third) administration of said drug and/or after at least the first (e.g. first, second, or third) administration of another form of therapeutic treatment (than the administration of a drug). It is particularly more preferred that the sample of exhaled breath is obtained from the patient in a time period of between 2 month and 2 weeks after at least the first (e.g. first, second, or third) administration of said drug and/or after at least the first (e.g. first, second, or third) administration of another form of therapeutic treatment (than the administration of a drug). For example, the sample of exhaled breath is obtained from the patient 1, 2, 3, 4, 5, 6 day(s), 1, 2, 3 week(s), 1, 2, or 3 month(s) after at least the first (e.g. first, second, or third) administration of said drug and/or after at least the first (e.g. first, second, or third) administration of another form of therapeutic treatment (than the administration of a drug).

In one embodiment, the level of the at least one compound is compared to a reference level of said at least one compound. Thus, in a particular embodiment, the present invention relates to a method of determining whether a patient (suffering from a MDD) responds to a therapeutic treatment of a major depressive disorder (MDD) which comprises the steps of:
(i) determining the level of at least one compound in a sample of exhaled breath obtained from a patient (suffering from a MDD), and
(ii) comparing the level of the at least one compound to a reference level of said at least one compound,
wherein the at least one compound is selected from the group consisting of methylguanidine, lactic acid, butyric acid, tetrahydro-tiaphene, putrescine, acetamide, isoprene, toluene, and acetonitrile.

The reference level may be any level that allows to determine whether the patient suffering from a MDD responds to the therapeutic treatment or not. It is preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one subject having a MDD, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 subject(s) having a MDD. It is more preferred that the reference level is the level determined by measuring between 2 and 500 reference samples of exhaled breath obtained from between 2 and 500 subjects having a MDD. It is even more preferred that the reference level is determined by measuring between 50 and 500 reference samples of exhaled breath obtained from between 50 and 500 subjects having a MDD. It is most preferred that the reference level is determined by measuring between 100 and 500 reference samples of exhaled breath obtained from between 100 and 500 subjects having a MDD.

It is also preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one healthy subject, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference samples of exhaled breath obtained from between 2 and 500 healthy subjects. It is even more preferred that the reference level is determined by measuring between 50 and 500 reference samples of exhaled breath obtained from between 50 and 500 healthy subjects. It is most preferred that the reference level is determined by measuring between 100 and 500 reference samples of exhaled breath obtained from between 100 and 500 healthy subjects.

It is practicable to take one reference sample of exhaled breath per subject for analysis. If additional reference samples of exhaled breath are required, such as to determine the reference level in different reference samples of exhaled breath (e.g. at different points in time), the same subject may be (re)tested. Said reference level may be an average reference level. It may be determined by measuring reference levels and calculating the "average" value (e.g. mean, median or modal value) thereof.

In a specific embodiment, the reference level represents a threshold which allows to divide/separate the patients in responders and non-responders.

In one alternative or additional embodiment, the reference level is the level determined in a reference sample of exhaled breath obtained from the (same) patient prior to the administration of said drug and/or prior to the administration of another form of therapeutic treatment (than the administration of a drug). It is particularly preferred that the reference sample of exhaled breath is obtained from the (same) patient in a time period of between 1 week and immediately prior to the administration of said drug and/or prior to the administration of another form of therapeutic treatment (than the administration of a drug). It is particularly more preferred that the reference sample of exhaled breath is obtained from the (same) patient in a time period of between 1 day and immediately prior to the administration of said drug and/or prior to the administration of another form of therapeutic treatment (than the administration of a drug). For example, the reference sample of exhaled breath is obtained from the (same) patient immediately, 10, 20, 30, 40, 50 minutes, 1, 2, 3, 4, 5, 6, 7, 8, 9, 10, 11, 12, 13, 14, 15, 16, 17, 18, 19, 20, 21, 22, 23 hour(s), 1, 2, 3, 4, 5, 6 days, or 1 week prior to the administration of said drug and/or prior to the administration of another form of therapeutic treatment (than the administration of a drug).

It is preferred (with respect to both embodiments described above) that
the level of the at least one compound selected from the group consisting of methylguanidine, lactic acid, butyric acid, tetrahydro-tiaphene, putrescine, acetamide, and isoprene above the reference level indicates that the patient responds to said treatment of a MDD.
In contrast thereto, a level of the at least one compound selected from the group consisting of methylguanidine, lactic acid, butyric acid, tetrahydro-tiaphene, putrescine, acetamide, and isoprene which is comparable (e.g. identical) with or lower than the reference level indicates that the patient does not respond to said treatment of a MDD.

It is further (additionally or alternatively) preferred (with respect to both embodiments described above) that the level of the at least one compound selected from the group consisting of toluene and acetonitrile below the reference level indicates that the patient responds to said treatment of a MDD.
In contrast thereto, a level of the at least one compound selected from the group consisting of toluene and acetonitrile which is comparable (e.g. identical) with or higher than the reference level indicates that the patient does not respond to said treatment of a MDD.

In particular, the level of the at least one compound is at least 0.5-fold, more preferably at least 1-fold, even more preferably at least 1.5-fold, still more preferably at least 2-fold, and most preferably at least 3-fold below/above the reference level. For example, the level of the at least one compound is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

It is specifically preferred that the level/reference level is determined in a sample of exhaled breath obtained from a patient (suffering from/having a MDD) at a point in time within 0 and 5 minutes after awakening (= at awakening or immediately after awakening), at a point in time within < 5 minutes and 30 minutes after awakening, or at a point in time within < 30 minutes and 60 minutes after awakening. In particular, the level/reference level is determined in a sample of exhaled breath obtained from a patient (suffering from/having a MDD) about 2 minutes (± 1 minute) after awakening, 30 minutes after awakening, or 60 minutes after awakening.

It is specifically more preferred that the level/reference level represents an average level of levels determined in a sample of exhaled breath obtained from a patient (suffering from/having a MDD) at a point in time within 0 and 5 minutes after awakening (= at awakening or immediately after awakening), at a point in time within < 5 minutes and 30 minutes after awakening, and at a point in time within < 30 minutes and 60 minutes after awakening (= baseline level). In particular, the level/reference level represents an average level of levels determined in a sample of exhaled breath obtained from a patient suffering from/having a MDD at awaking, 30 minutes after awakening, and 60 minutes after awakening (= baseline level).

If the treatment encompasses the administration of a drug and the patient responds to said treatment, the drug administration may be continued, the dose of the drug may be reduced, or the drug administration may be stopped. If the treatment encompasses the administration of a drug and the patient does not respond to said treatment, the dose of the drug may be increased, the drug may be changed, or the therapy mode may be changed.

It is also (additionally or alternatively) preferred that the drug to be used in said treatment is an antidepressant, preferably an antidepressant selected from the group consisting of citalopram, escitalopram, paroxetine, sertraline, fluoxetine, venlafaxine, duloxetine, milnaciprame, bupropion, and mirtazapine, and/or that the other form of therapeutic treatment is selected from the group consisting of psychotherapy, exercise training, cognitive training, and physical rehabilitation.

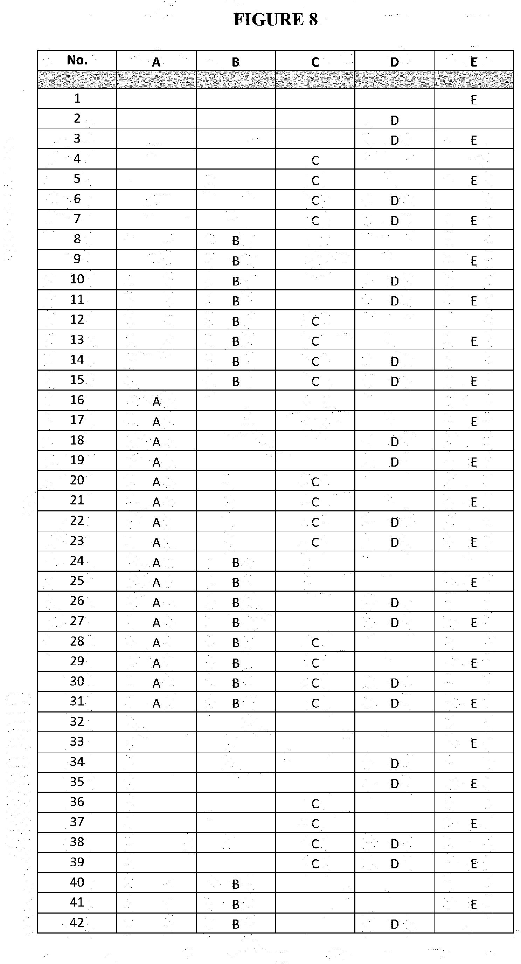

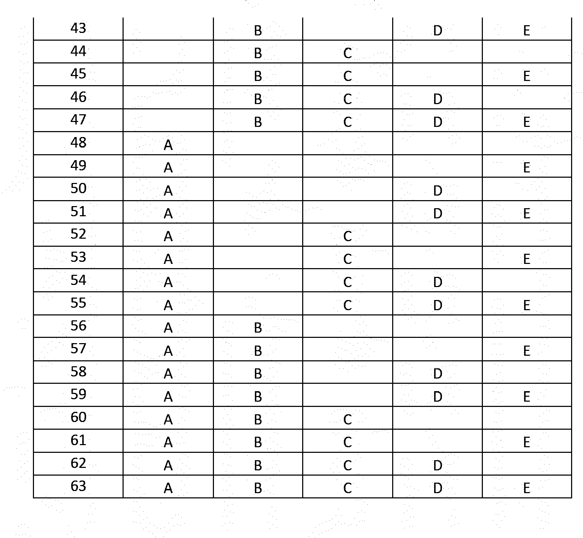

The following applies to the first to third aspect of the present invention:
As mentioned above, the at least one compound is selected from the group consisting of methylguanidine, lactic acid, butyric acid, tetrahydro-tiaphene, putrescine, acetamide, isoprene, toluene, and acetonitrile. In the first to third aspect of the present invention, the at least one compound is preferably selected from the group consisting of lactic acid, butyric acid, tetrahydro-tiaphene, putrescine, and acetamide. More preferably, the level of the following compounds: lactic acid, butyric acid, tetrahydro-tiaphene, putrescine, and acetamide is determined in a sample of exhaled breath obtained from a patient. **Figure 8** includes further combination of compounds which are particularly preferred.

In the first to third aspect of the present invention, the acetamide is preferably further selected from the group consisting of Hydroxy-*N*-methyl acetamide, *N*-Methoxy acetamide, *N-*ethyl-acetamide, and *N,N*-dimethyl-acetamide.

Diverse forms of a major depressive disorder (MDD) are known. In the first to third aspect of the present invention, the MDD is preferably selected from the group consisting of melancholic depression, atypical depression, catatonic depression, depression with anxious distress, depression with peri-partum onset, post-partum onset, and seasonal affective disorder.

The patient may be any individual suffering from a MDD. In the first to third aspect of the present invention, the patient is preferably a mammal, more preferably a human.

The level of the at least one compound selected from the group consisting of methylguanidine, lactic acid, butyric acid, tetrahydro-tiaphene, putrescine, acetamide, isoprene, toluene, and acetonitrile may be determined by any suitable method allowing the detection and quantification of said compound. The level of the at least one compound selected from the group consisting of methylguanidine, lactic acid, butyric acid, tetrahydro-tiaphene, putrescine, acetamide, isoprene, toluene, and acetonitrile is preferably determined by spectrometry or chromatography.
Suitable techniques include all chromatographic separation techniques such as liquid chromatography (LC), high performance liquid chromatography (HPLC), gas chromatography (GC), thin layer chromatography, or size exclusion or affinity chromatography. These techniques are well known in the art and can be applied by the person skilled in the art without further ado. Suitable devices for such determination are also well known in the art.
Suitable techniques also include all mass spectrometry techniques such as time of flight mass spectrometry (TOF-MS), proton-transfer-reaction mass spectrometry (PTR-MS), quadrupole mass spectrometry (QMS), gas chromatography mass spectrometry (GC-MS), liquid chromatography mass spectrometry (LC-MS), direct infusion mass spectrometry, fourier transform ion-cyclotrone-resonance mass spectrometry (FT-ICR-MS), capillary electrophoresis mass spectrometry (CE-MS), any sequentially coupled mass spectrometry, such as MS-MS or MS-MS-MS, inductively coupled plasma mass spectrometry (ICP-MS), pyrolysis mass spectrometry (Py-MS), or ion mobility mass spectrometry.
As an alternative or in addition to mass spectrometry techniques, the following techniques may be used for determination: nuclear magnetic resonance (NMR), magnetic resonance imaging (MRI), Fourier transform infrared analysis (FT-IR), ultraviolet (UV) spectroscopy, refraction index (Rl), fluorescent detection, radiochemical detection, electrochemical detection, light scattering (LS), dispersive Raman spectroscopy or flame ionisation detection (FID). These techniques are well known to the person skilled in the art and can be applied without further ado.
More preferably (i) the spectrometry is mass spectrometry (MS), and/or (ii) the chromatography is liquid chromatography (LC) or affinity chromatography. Even more preferably, the mass spectrometry (MS) is selected from the group consisting of Proton-Transfer-Reaction mass spectrometry (PTR-MS), Time of Flight mass spectrometry (TOF-MS), and quadrupole mass spectrometry (QMS).

All methods of the present invention may also be designated as *in vitro* methods.

In a fourth aspect, the present invention relates to the use of at least one compound for diagnosing a major depressive disorder (MDD) in a patient (suspected of having a MDD), for determining the course of a major depressive disorder (MDD) in a patient (having a MDD), or for determining whether a patient (suffering from a MDD) responds to a therapeutic treatment of a MDD, wherein the at least one compound is selected from the group consisting of methylguanidine, lactic acid, butyric acid, tetrahydro-tiaphene, putrescine, acetamide, isoprene, toluene, and acetonitrile.
The diagnosing of a MDD allows to determine whether a patient suffers from a MDD or not.
The determining of the course of a MDD allows to determine whether a MDD worsens in the patient, does not worsen/is stable/not progressing in the patient, or improves in the patient. The determining whether a patient responds to a therapeutic treatment of a MDD allows to examine, whether the therapeutic treatment of a MDD is effective or not.

In order to diagnose a MDD in a patient, to determine the course of a MDD in a patient, or to determine whether a patient responds to a therapeutic treatment of a MDD, the level of the at least one compound selected from the group consisting of methylguanidine, lactic acid, butyric acid, tetrahydro-tiaphene, putrescine, acetamide, isoprene, toluene, and acetonitrile is determined in a sample of exhaled breath of said patient.

The at least one compound is preferably selected from the group consisting of lactic acid, butyric acid, tetrahydro-tiaphene, putrescine, and acetamide. More preferably, the following compounds are used: lactic acid, butyric acid, tetrahydro-tiaphene, putrescine, and acetamide. **Figure 8** includes further combinations of compounds which are particularly preferred.

The acetamide is preferably further selected from the group consisting of Hydroxy-*N-*methyl acetamide, *N*-Methoxy acetamide, *N*-ethyl-acetamide, *N,N*-dimethyl-acetamide.

Diverse forms of a major depressive disorder (MDD) are known. The MDD is preferably selected from the group consisting of melancholic depression, atypical depression, catatonic depression, depression with anxious distress, depression with peri-partum onset, post-partum onset, and seasonal affective disorder.

The patient may be any individual suffering from a MDD. The patient is preferably a mammal, more preferably a human.

In a fifth aspect, the present invention relates to a device comprising
(i) an inlet for receiving exhaled breath, and
(ii) an analysis unit configured to analyze the exhaled breath,
wherein the analysis comprises the determination of the level of at least one compound in a sample of exhaled breath obtained from a patient, and
wherein the at least one compound is selected from the group consisting of methylguanidine, lactic acid, butyric acid, tetrahydro-tiaphene, putrescine, acetamide, isoprene, toluene, and acetonitrile.

The device is capable of performing the methods of the first to third aspect.

In one preferred embodiment, the device further comprises an output unit which is configured to provide an indication to the user which is characteristic for the result of the analysis.

In one more preferred embodiment, the device is a handheld device or a portable device.

In a sixth aspect, the present invention relates to a kit comprising the device of the fifth aspect.

In one preferred embodiment, the kit comprises instructions on how to carry out the methods of the first to third aspect.

In one more preferred embodiment, the kit is useful for conducting the methods of the first to third aspect.

The invention is summarized as follows:
1. A method of diagnosing a major depressive disorder (MDD) in a patient (suspected of having a MDD) comprising the step of:
   determining the level of at least one compound in a sample of exhaled breath obtained from a patient (suspected of having a MDD),
   wherein the at least one compound is selected from the group consisting of methylguanidine, lactic acid, butyric acid, tetrahydro-tiaphene, putrescine, acetamide, isoprene, toluene, and acetonitrile.
2. The method of item 1, wherein the level of the at least one compound is compared to a reference level of said at least one compound.
3. The method of item 2, wherein the reference level is the level determined by measuring at least one reference sample of exhaled breath obtained from at least one healthy subject.
4. The method of item 3, wherein
   the level of the at least one compound selected from the group consisting of methylguanidine, lactic acid, butyric acid, tetrahydro-tiaphene, putrescine, acetamide, and isoprene below the reference level indicates that the patient has a MDD, and/or the level of the at least one compound selected from the group consisting of toluene and acetonitrile above the reference level indicates that the patient has a MDD.
5. A method of determining the course of a major depressive disorder (MDD) in a patient (having a MDD) comprising the step of:
   determining the level of at least one compound in a sample of exhaled breath obtained from a patient (having a MDD),
   wherein the at least one compound is selected from the group consisting of methylguanidine, lactic acid, butyric acid, tetrahydro-tiaphene, putrescine, acetamide, isoprene, toluene, and acetonitrile.
6. The method of item 5, wherein the level of the at least one compound is compared to a reference level of said at least one compound.
7. The method of item 6, wherein the reference level is the level determined by measuring at least one reference sample of exhaled breath obtained from at least one subject having a MDD.
8. The method of any one of items 5 to 7, wherein said determining comprises determining the level of the at least one compound in a sample of exhaled breath obtained from a patient (having a MDD) at a first point in time and in at least one further sample of exhaled breath obtained from the (same) patient at a later point in time and comparing said levels determined at the different time points.
9. The method of item 8, wherein the level of the compound selected from the group consisting of methylguanidine, lactic acid, butyric acid, tetrahydro-tiaphene, putrescine, acetamide, and isoprene which
   (i) decreases over time indicates that the MDD worsens in the patient,
   (ii) does not change over time indicates that the MDD does not worsen/is stable in the patient, or
   (iii) increases over time indicates that the MDD improves in the patient.
10. The method of items 8 or 9, wherein the level of the compound selected from the group consisting of toluene and acetonitrile which
   (i) increases over time indicates that the MDD worsens in the patient,
   (ii) does not change over time indicates that the MDD does not worsen/is stable in the patient, or
   (iii) decreases over time indicates that the MDD improves in the patient.
11. The method of any one of items 5 to 10, wherein the patient receives, has received, or had received a treatment of a MDD.
12. The method of item 11, wherein the treatment of a MDD is selected from the group consisting of the administration of a drug, psychotherapy, exercise training, cognitive training, and physical rehabilitation.
13. A method of determining whether a patient (suffering from a MDD) responds to a therapeutic treatment of a major depressive disorder (MDD) comprising the step of:
   determining the level of at least one compound in a sample of exhaled breath obtained from a patient (suffering from a MDD),
   wherein the at least one compound is selected from the group consisting of methylguanidine, lactic acid, butyric acid, tetrahydro-tiaphene, putrescine, acetamide, isoprene, toluene, and acetonitrile.
14. The method of item 13, wherein the patient is a patient to whom at least once
   a drug to be used in said therapeutic treatment, and/or
   another form of therapeutic treatment
   is administered, has been administered, or had been administered.
15. The method of item 14, wherein the sample of exhaled breath is obtained from the patient after at least the first
   administration of said drug and/or
   administration of another form of therapeutic treatment.
16. The method of any one of items 13 to 15, wherein the level of the at least one compound is compared to a reference level of said at least one compound.
17. The method of item 16, wherein the reference level is the level determined by measuring at least one reference sample of exhaled breath obtained from at least one subject having a MDD.
18. The method of items 16 or 17, wherein the reference level is the level determined in a reference sample of exhaled breath obtained from the (same) patient prior to the administration of said drug, and/or
   administration of another form of therapeutic treatment.
19. The method of any one of items 16 to 18, wherein
   the level of the at least one compound selected from the group consisting of methylguanidine, lactic acid, butyric acid, tetrahydro-tiaphene, putrescine, acetamide, and isoprene above the reference level indicates that the patient responds to said treatment of a MDD, and/or
   the level of the at least one compound selected from the group consisting of toluene and acetonitrile below the reference level indicates that the patient responds to said treatment of a MDD.
20. The method of any one of items 14 to 19, wherein
   the drug to be used in said treatment is an antidepressant, preferably an antidepressant selected from the group consisting of citalopram, escitalopram, paroxetine, sertraline, fluoxetine, venlafaxine, duloxetine, milnaciprame, bupropion, and mirtazapine, and/or the other form of therapeutic treatment is selected from the group consisting of psychotherapy, exercise training, cognitive training, and physical rehabilitation.
21. The method of any one of items 1 to 20, wherein the acetamide is selected from the group consisting of Hydroxy-*N*-methyl acetamide, *N*-Methoxy acetamide, *N*-ethyl-acetamide, *N,N*-dimethyl-acetamide.
22. The method of any one of items 1 to 21, wherein the MDD is selected from the group consisting of melancholic depression, atypical depression, catatonic depression, depression with anxious distress, depression with peri-partum onset, post-partum onset, and seasonal affective disorder.
23. The method of any one of items 1 to 22, wherein the patient is a mammal, preferably a human.
24. The method of any one of items 1 to 23, wherein the level of the at least one compound is determined by spectrometry or chromatography.
25. The method of item 24, wherein
   (i) the spectrometry is mass spectrometry (MS), and/or
   (ii) the chromatography is liquid chromatography (LC) or affinity chromatography.
26. The method of item 25, wherein the mass spectrometry (MS) is selected from the group consisting of Proton-Transfer-Reaction mass spectrometry (PTR-MS), Time of Flight mass spectrometry (TOF-MS), and quadrupole mass spectrometry (QMS).
27. Use of at least one compound for diagnosing a major depressive disorder (MDD) in a patient (suspected of having a MDD), for determining the course of a major depressive disorder (MDD) in a patient (having a MDD), or for determining whether a patient (suffering from a MDD) responds to a therapeutic treatment of a MDD, wherein the at least one compound is selected from the group consisting of methylguanidine, lactic acid, butyric acid, tetrahydro-tiaphene, putrescine, acetamide, isoprene, toluene, and acetonitrile.
28. A device comprising
   (i) an inlet for receiving exhaled breath, and
   (ii) an analysis unit configured to analyze the exhaled breath,
      wherein the analysis comprises the determination of the level of at least one compound in a sample of exhaled breath obtained from a patient, and
      wherein the at least one compound is selected from the group consisting of methylguanidine, lactic acid, butyric acid, tetrahydro-tiaphene, putrescine, acetamide, isoprene, toluene, and acetonitrile.
29. The device of item 28, wherein the device further comprises an output unit which is configured to provide an indication to the user which is characteristic for the result of the analysis.
30. The device of items 28 or 29, wherein the device is a handheld device or portable device.
31. The device of any one of items 28 to 30, wherein the device is capable of performing the methods of any one of items 1 to 26.
32. A kit comprising the device of any one of items 28 to 31.
33. The kit of item 32, wherein the kit comprises instructions on how to carry out the methods of any one of items 1 to 26.
34. The kit of items 32 or 33, wherein the kit is useful for conducting the methods of any one of items 1 to 26.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following figures and examples are merely illustrative of the present invention and should not be construed to limit the scope of the invention as indicated by the appended claims in any way.
**Figure 1****:** Shows that the marker with mass 74 (methylguanidine) was decreased in exhaled breath of MDD patients compared to healthy controls
**Figure 2****:** Shows that the marker with mass 88 (butyric acid) was decreased in exhaled breath of MDD patients compared to healthy controls.
**Figure 3****:** Shows that the marker with mass 89 (tetrahydro-tiaphene/putrescine) was decreased in exhaled breath of MDD patients compared to healthy controls.
**Figure 4****:** Shows that the marker with mass 90 (lactic acid/acetamides) was decreased in exhaled breath of MDD patients compared to healthy controls.
**Figure 5****:** Shows that the marker with mass 69 (isoprene) was decreased in exhaled breath of MDD patients compared to healthy controls.
**Figure 6****:** Shows that the marker with mass 42 (acetonitrile) was increased in exhaled breath of MDD patients compared to healthy controls.
**Figure 7****:** Shows that the marker with mass 93 (toluene) was increased in exhaled breath of MDD patients compared to healthy controls.
**Figure 8****:** Shows combinations of compounds which are particularly preferred. A = methylguanidine, B = lactic acid, C = butyric acid, D = tetrahydro-tiaphene, E = putrescine.
**Figure 9****:** Shows the ROC curve for mass 74 (methylguanidine) in the confirmation sample (AUC 0.83)
**Figure 10****:** Shows the ROC curve for masses 69 (isoprene), 74 (methylguanidine), 88 (butyric acid) and 93 (toluene) in the confirmation sample (AUC 0.86)

### EXAMPLES

The examples given below are for illustrative purposes only and do not limit the invention described above in any way.

### 1. Materials & Methods

### 1.1 Participants

### Participant selection

16 patients with major depressive disorder (MDD) diagnosis according to the Diagnostic and Statistical Manual of Mental Disorders-V (DSM-V) and 16 healthy controls were recruited as test sample. The 16 patients with MDD according to the DSM-V were recruited from the psychiatric service and hospital. The healthy controls were recruited from the local community. Moreover, 10 depressed patients with DSM-V MDD diagnosis and 9 healthy controls were recruited in a second phase as validation sample.

### Inclusion criteria

Inclusion criteria for patients were MDD according to DSM-V and age between 18 and 65 years. The latter applied to healthy controls too. Patients were managed in accordance to S3 treatment guidelines for MDD. All patients and controls gave written informed consent to participate in the study.

### Exclusion criteria

Exclusion criteria for patients were a current psychotic disorder, depressive episodes with psychotic symptoms, and suicidal intend. Moreover, subjects with a known alcohol or drug dependency were excluded. Neurological disorders and diseases affecting the brain function were further excluded (e.g. hypo- or hyperthyreodism). Patients with comorbid personality disorders were also excluded. Other independent psychiatric axis 1 disorders were excluded. The healthy controls recruited from the local community were matched for age, sex and education to the patients included into the study.

### Hormonal cycle

Hormonal cycle and contraceptiva in women were assessed by questionnaires.

### Nutrition and Smoking

The type of diet can have an impact on metabolites excreted and thus these effects can be seen in exhaled breath. Thus, nutrition history was taken. Breath gas analysis was carried out in patients and controls at 8 a.m. and onwards on an empty stomach.
Smoking also influences some of volatile organic compounds (VOCs). In smokers benzene, xylene isomers, styrene, ethylbenzene, acetonitrile and furan derivatives as well as hydrocarbons have been detected in exhaled breath (Buszewski *et al.*, 2009). With regards to smoking this will be documented and moreover, it is clearly detected with breath gas analysis and the VOCs concerned can be excluded from the analysis.

### 1.2 Clinical assessments

All subjects were investigated clinically using the Mini-International-Neuropsychiatric Interview (MINI) to document and verify the clinical diagnoses. Moreover, they have been examined using standardized questionnaires for psychiatric symptoms: Montgomery Asberg Rating Scale (MADRS), Hamilton Anxiety Rating Scale (HARS), Clinical Global Impression (CGI) and Beck Depression Inventory (BDI). In addition to the clinical interview, the current general medical conditions was assessed by the 14-item Cumulative Illness Rating Scale (CIRS) and occupational and interpersonal adjustment using the Work and Social Adjustment Scale.
The current medication and also the medication during the last two weeks before study inclusion were documented for the group of patients receiving antidepressants.
Furthermore, the past clinical course was assessed from interviews and case notes within the psychiatric services in particular to stage the recurrence of the disease. Moreover, previous other doctors' reports were collected. Thus, also exact as possible treatment history for medications and psychotherapy were obtained.

### 1.3 Breath gas analysis

The concentrations of the volatile organic compounds (VOCs) in the breath gas of all participants (patients as well as controls) were measured with proton-transfer-reaction mass spectrometry (PTR-MS). Breath gas probes can be taken at awakening, after 30 min, and/or after 60 min at baseline and at the two week and four week follow-ups. This study was performed with a standard PTR-MS system using a quadrupole as the analyzer unit.
For better detection of the exact substances, 6 probes were analyzed with the PTR-MS system as well as with a more sophisticated system based on a time-of-flight (TOF) analyzer unit. The sensitivity and specificity of such a system is by far better than the quadrupole based system. The general concept of PTR-MS is to bring the breath gas into a drift tube. Here, the gas will be mixed and thus react with protons (H₃O⁺ - ions). The protonated VOCs will then be analyzed by the mass spectrometry part. Typically masses between m/z 19 and 200 can be analyzed with the quadrupole based analyzer unit and between m/z 19 and about 800 with the TOF based analyzer. The linear range of the system is between a few volume parts per trillion (pptv) and a few hundred parts per billion (ppbv). With the TOF based analyzer, the mass resolution can also be increased by at least one order of magnitude allowing better identification of the identified VOCs.
The breath gas was collected with a dedicated breath gas sampler. This sampler improves the sensitivity and accuracy even further and might help to improve the timing.
No further preparation like freezing or making use of condensation is necessary when working with the PTR-MS system. It can directly be worked with the exhaled breath.

### 1.4 Methods for data analysis

In the test sample, differences between patients and healthy controls were analyzed using cliffs delta statistics which are independent from distribution of the data. Markers to be used in random forest analyses (A) and logistic regression analyses (B) were selected based on the cliffs delta statistics. Random forest analyses were run with inputs as a function of cliffs delta statistics in order to identify the optimal number of markers relevant for classification. First, these were done for breath gas markers only, secondly demography data such as age, gender, history of smoking and alcohol were further included. Sensitivity, specificity and accuracy are given for discrimination between patients with MDD and health controls, for significant different markers.
The results were confirmed in the validation sample.

Moreover, in the test and validation samples discrimination analysis were carried out, in order to obtain sensitivity, specificity and predictive power of the markers.

### 2. Results

### 2.1 Classification Analysis

For this purpose, all test persons were divided into 2 groups. The first 16 patients with MDD and 16 matched healthy controls were the test sample. The remaining other 19 participants (10 patients with MDD and 9 healthy controls) were the confirmation sample.

Per definition, patients with MDD show significant higher depression severity compared to healthy controls. There were no significant differences between the Test Sample and the Confirmation Sample in terms of gender, age, BMI and the severity of the illness (see **Table 1**).

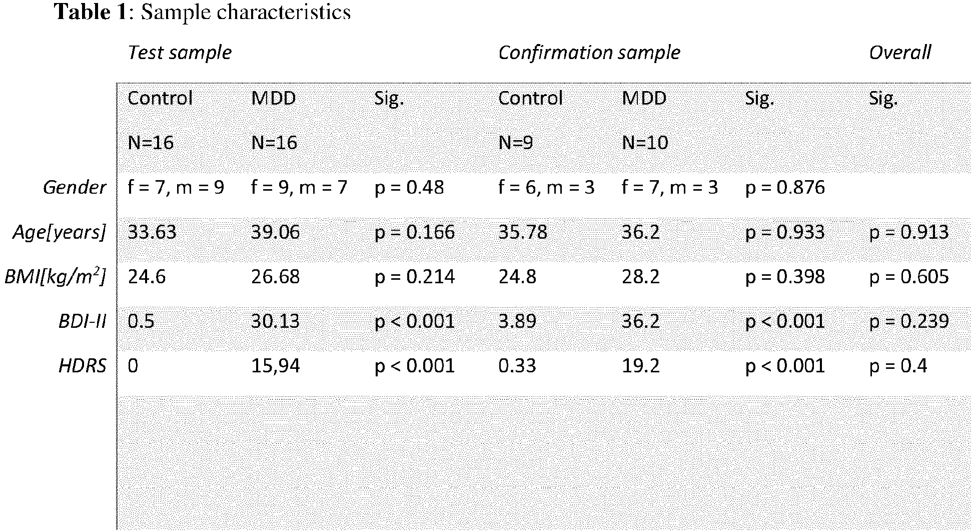

### 2.2 Machine learning using random forest analysis

Using Cliffs Delta statistics the differences based on effect sizes irrespective of distribution of values were used.

The following markers were (significantly) deregulated between patients with MDD and healthy controls: methylguanidine (74 m/z), lactic acid (90 m/z), butyric acid (88 m/z), tetrahydro-tiaphene (89 m/z), putrescine (89 m/z), acetamide (90 m/z), isoprene (69 m/z), toluene (93 m/z), and acetonitrile (42 m/z). The data are summarized in **Table 2** below.

**Table 2: Summary of the markers significantly deregulated between patients with MDD and healthy controls**

| Mass | Patients with MDD | Healty Controls | F (1/50) | p-value | pFDR corrected |
|---|---|---|---|---|---|
| 42 bl | 111.1 ±143.8 | 30.7 ±36 | 7.6 | 0.00847 | 0.09317 |
| 42 30min | 114.2 ±166 | 24.2 ±16.5 | 7.6 | 0.00841 | 0.09317 |
| 42 60min | 120.5 ±180.8 | 17.7 ±11.9 | 8.4 | 0.00565 | 0.09317 |
| 69 30min | 197.8 ±124.7 | 288 ±170.5 | 4.5 | 0.03926 | 0.24915 |
| 74 30min | 18.8 ±13.2 | 35 ±30.7 | 4.9 | 0.03146 | 0.21629 |
| 74 60min | 17.9 ±11.5 | 32.6 ±12.3 | 18 | 0.0001 | 0.0165 |
| 88 bl | 2908.3 ±3260.7 | 6887.4 ±5864.5 | 9.2 | 0.0031 | 0.09317 |
| 88 30min | 3669.6 ±4038.8 | 8010.5 ± 7012.3 | 8.3 | 0.00607 | 0.09317 |
| 88 60min | 3686.6 ±4261.5 | 8453 ±7810.3 | 8 | 0.00692 | 0.09317 |
| 89 bl | 162.6 ±178.1 | 336.6 ±273.9 | 8.1 | 0.00631 | 0.09317 |
| 89 30 min | 192.5 ±198.2 | 403.8 ±356.2 | 7.8 | 0.00739 | 0.09317 |
| 89 60min | 181.8 ±199.1 | 404.9 ±371 | 7.8 | 0.00743 | 0.09317 |
| 90 bl | 9.1 ±8.9 | 21.9 ±19.8 | 9.6 | 0.00323 | 0.09317 |
| 90 30min | 12 ±13.8 | 24.2 ±22.1 | 6.3 | 0.01539 | 0.15685 |
| 90 60min | 11 ±12.6 | 26.6 ±27.2 | 7.6 | 0.00796 | 0.09317 |
| 93 30min | 3.9 ±3.9 | 0.8 ±1.6 | 12.8 | 0.0082 | 0.06765 |

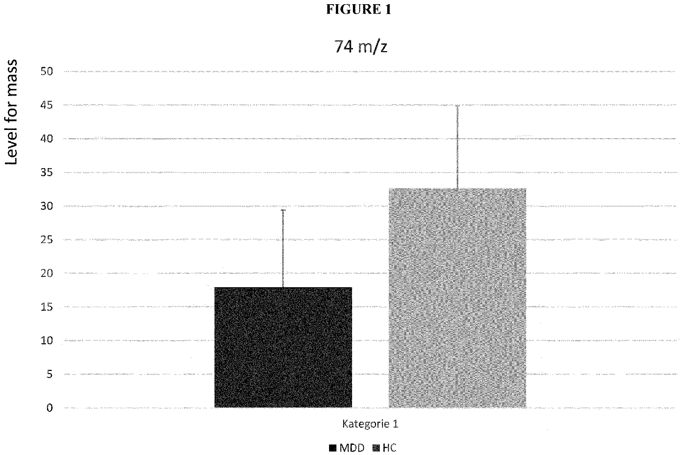

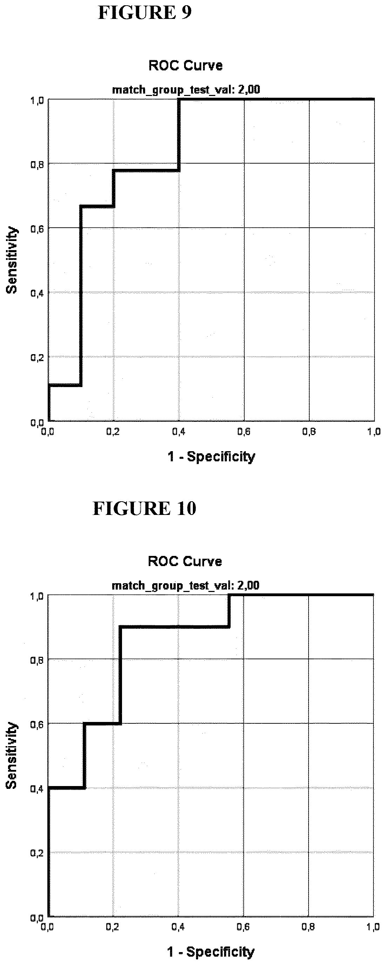

The concentration of mass 74 (methylguanidine) showed decreased levels in patients with MDD compared to healthy controls (see **Table 2** and **Figure 1**). Prediction accuracy was high for this marker even in the confirmation sample (**Figure 9**).

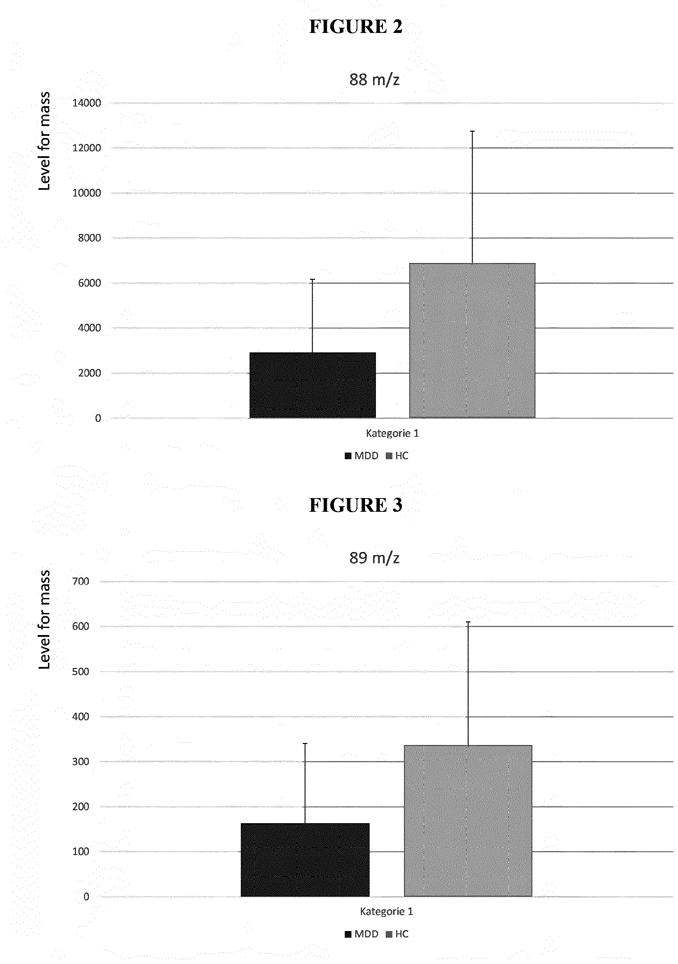

The concentrations of masses 88 (butyric acid), 89 (tetrahydro-tiaphene/putrescine), and 90 (lactic acid/acetamides) showed decreased levels in patients with MDD compared to healthy controls. No significant time effects or time x group interactions were found for these masses (see **Table 2** and **Figures 2, 3****,** and **4**).

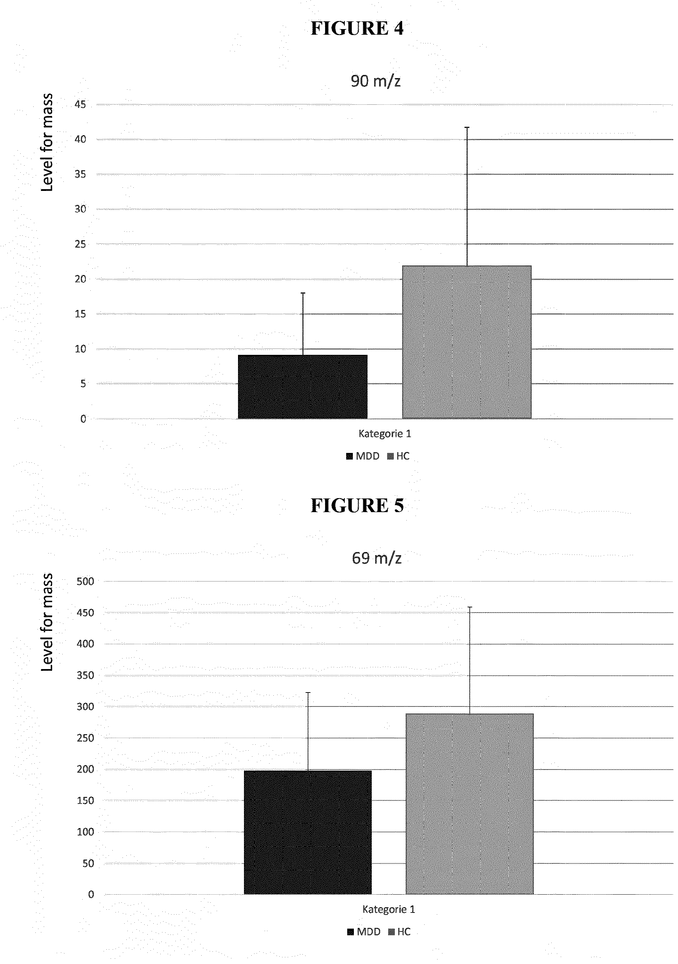

The concentration of mass 69 (isoprene) showed decreased levels in patients with MDD compared to healthy controls (see **Table 2** and **Figure 5**).

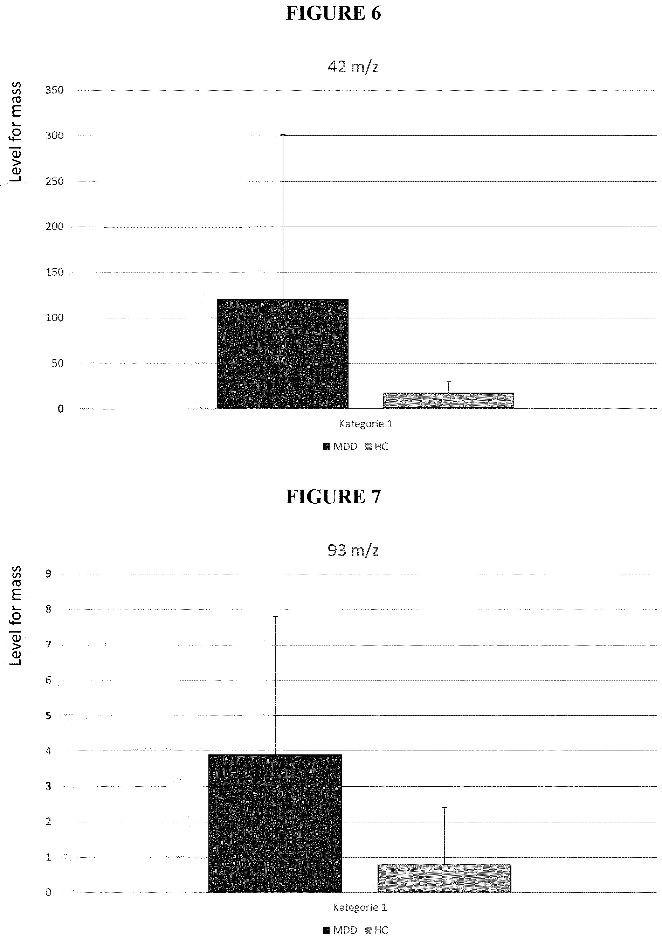

The concentration of mass 42 (acetonitrile) showed increased levels in patients with MDD compared to healthy controls (see **Table 2** and **Figure 6**).

The concentration of mass 93 (toluene) showed increased levels in patients with MDD compared to healthy controls (see **Table 2** and **Figure 7**).

Taking breath gas markers from baseline measurements at awakening, 30 min after awakening, or 60 minutes after awakening resulted in good accuracy, e.g. an accuracy of 80 % after awakening.

Marker combinations also improved accuracy (see **Figure 8**). The use of the markers mass 69 (isoprene), mass 74 (methylguanidine), mass 88 (butyric acid), and mass 93 (toluene) in the confirmation sample showed very good accuracy (AUC 0.86) (**Figure 10**).

### 3. Discussion

In the factor solution, lactic acid (90m/z), butyric acid (88 m/z, 89 m/z), Tetrahydro-tiaphene (89 m/z), Putrescine (89 m/z) and Acetamides (Hydroxy-*N*-methyl acetamide, *N*-Methoxy acetamide, *N*-ethyl-acetamide, *N,N*-dimethyl-acetamide (90 m/z)) were included.

Although glucose is usually assumed to be the main energy source for living tissues, there are some indications that it is lactate, and not glucose, that is preferentially metabolized by neurons in the brain of several mammalian species. Lactic acid is downregulated in MDD patients compared to healthy controls indicating a disturbed lactate metabolism.

Butyric acid plays and important role in oxidative generation of ATP, supplies anabolic pathways (gluconeogenesis and lipogenesis) with carbon-containing precursor molecules and contributes to the regulation of cell metabolism by triggering signaling pathways. Butyric acid is found to be reduced in major depression in this study indicating a disturbed energy metabolism which fits very well to the symptoms of depression.

Tetrahydro-tiaphene is a core structure of Biotin (Vitamin H), which is involved in energy metabolism as well. Reduction of biotin is associated with depression. It also plays an important role in epigenetic regulation. Acetamides play a role in metabolism as well. Putrescine is a fermentation product.

Isoprene (69m/z) is involved in cholesterol biosynthesis and also plays a role in energy metabolism, but with negative metabolism and is associated with risk for cardiovascular diseases. Interestingly, cardiovascular diseases were found to be associated with depression. It is altered in depression in this study and earlier was found to be associated with sleep disorders, chronic liver disease and kidney disease.

Acetonitrile (42 m/z) and toluene (93 m/z), which are increased in patients with depression compared to healthy controls, are markers for toxicity from the environment.

### REFERENCES

Kennis, M., Gerritsen, L., van Dalen, M., Williams, A., Cuijpers, P. & Bockting, C. (2020). Prospective biomarkers of major depressive disorder: a systematic review and meta-analysis. Mol Psychiatry 25, 321-338.
Kudielka, B. M., Hawkley, L. C., Adam, E. K. & Cacioppo, J. T. (2007). Compliance with ambulatory saliva sampling in the chicago health, aging, and social relations study and associations with social support. Ann Behav Med 34, 209-16.
Buszewski, B., Ulanowska, A., Ligor, T., Denderz, N. & Amann, A. (2009). Analysis of exhaled breath from smokers, passive smokers and non-smokers by solid-phase microextraction gas chromatography/mass spectrometry. Biomed Chromatogr 23, 551-6.

## Claims

1. A method of diagnosing a major depressive disorder (MDD) in a patient (suspected of having a MDD) comprising the step of:
determining the level of at least one compound in a sample of exhaled breath obtained from a patient (suspected of having a MDD),
wherein the at least one compound is selected from the group consisting of methylguanidine, lactic acid, butyric acid, tetrahydro-tiaphene, putrescine, acetamide, isoprene, toluene, and acetonitrile.

2. The method of claim 1,
wherein the level of the at least one compound is compared to a reference level of said at least one compound,
wherein the reference level is preferably the level determined by measuring at least one reference sample of exhaled breath obtained from at least one healthy subject, wherein more preferably
the level of the at least one compound selected from the group consisting of methylguanidine, lactic acid, butyric acid, tetrahydro-tiaphene, putrescine, acetamide, and isoprene below the reference level indicates that the patient has a MDD, and/or the level of the at least one compound selected from the group consisting of toluene and acetonitrile above the reference level indicates that the patient has a MDD.

3. A method of determining the course of a major depressive disorder (MDD) in a patient (having a MDD) comprising the step of:
determining the level of at least one compound in a sample of exhaled breath obtained from a patient (having a MDD),
wherein the at least one compound is selected from the group consisting of methylguanidine, lactic acid, butyric acid, tetrahydro-tiaphene, putrescine, acetamide, isoprene, toluene, and acetonitrile.

4. The method of claim 3,
wherein said determining comprises determining the level of the at least one compound in a sample of exhaled breath obtained from a patient (having a MDD) at a first point in time and in at least one further sample of exhaled breath obtained from the (same) patient at a later point in time and comparing said levels determined at the different time points,
wherein preferably the level of the compound selected from the group consisting of methylguanidine, lactic acid, butyric acid, tetrahydro-tiaphene, putrescine, acetamide, and isoprene which
(i) decreases over time indicates that the MDD worsens in the patient,
(ii) does not change over time indicates that the MDD does not worsen/is stable in the patient, or
(iii) increases over time indicates that the MDD improves in the patient, or
wherein preferably the level of the compound selected from the group consisting of toluene and acetonitrile which
(i) increases over time indicates that the MDD worsens in the patient,
(ii) does not change over time indicates that the MDD does not worsen/is stable in the patient, or
(iii) decreases over time indicates that the MDD improves in the patient.

5. The method of any one of claims 3 or 4,
wherein the patient receives, has received, or had received a treatment of a MDD, wherein the treatment of a MDD is preferably selected from the group consisting of the administration of a drug, psychotherapy, exercise training, cognitive training, and physical rehabilitation.

6. A method of determining whether a patient (suffering from a MDD) responds to a therapeutic treatment of a major depressive disorder (MDD) comprising the step of:
determining the level of at least one compound in a sample of exhaled breath obtained from a patient (suffering from a MDD),
wherein the at least one compound is selected from the group consisting of methylguanidine, lactic acid, butyric acid, tetrahydro-tiaphene, putrescine, acetamide, isoprene, toluene, and acetonitrile.

7. The method of claim 6, wherein the patient is a patient to whom at least once a drug to be used in said therapeutic treatment, and/or
another form of therapeutic treatment
is administered, has been administered, or had been administered.

8. The method of claim 7, wherein the sample of exhaled breath is obtained from the patient after at least the first
administration of said drug and/or
administration of another form of therapeutic treatment.

9. The method of any one of claims 6 to 8, wherein the level of the at least one compound is compared to a reference level of said at least one compound.

10. The method of claim 9, wherein the reference level is the level determined by measuring at least one reference sample of exhaled breath obtained from at least one subject having a MDD.

11. The method of claims 9 or 10, wherein the reference level is the level determined in a reference sample of exhaled breath obtained from the (same) patient prior to the administration of said drug, and/or
administration of another form of therapeutic treatment.

12. The method of any one of claims 9 to 11, wherein
the level of the at least one compound selected from the group consisting of methylguanidine, lactic acid, butyric acid, tetrahydro-tiaphene, putrescine, acetamide, and isoprene above the reference level indicates that the patient responds to said treatment of a MDD, and/or
the level of the at least one compound selected from the group consisting of toluene and acetonitrile below the reference level indicates that the patient responds to said treatment of a MDD.

13. Use of at least one compound for diagnosing a major depressive disorder (MDD) in a patient (suspected of having a MDD), for determining the course of a major depressive disorder (MDD) in a patient (having a MDD), or for determining whether a patient (suffering from a MDD) responds to a therapeutic treatment of a MDD, wherein the at least one compound is selected from the group consisting of methylguanidine, lactic acid, butyric acid, tetrahydro-tiaphene, putrescine, acetamide, isoprene, toluene, and acetonitrile.

14. A device comprising
(i) an inlet for receiving exhaled breath, and
(ii) an analysis unit configured to analyze the exhaled breath,
wherein the analysis comprises the determination of the level of at least one compound in a sample of exhaled breath obtained from a patient, and
wherein the at least one compound is selected from the group consisting of methylguanidine, lactic acid, butyric acid, tetrahydro-tiaphene, putrescine, acetamide, isoprene, toluene, and acetonitrile.

15. A kit comprising the device of claim 14.
